(19) Europäisches Patentamt
European Patent Office
Office européen des brevets

(11) Numéro de publication: **0 113 632**
**A1**

(12) **DEMANDE DE BREVET EUROPEEN**

(21) Numéro de dépôt: **83402535.5**

(22) Date de dépôt: **26.12.83**

(51) Int. Cl.³: **C 07 D 221/28**
A 61 K 31/435

(30) Priorité: **30.12.82 FR 8222133**

(43) Date de publication de la demande:
**18.07.84 Bulletin 84/29**

(84) Etats contractants désignés:
**AT BE CH DE FR GB IT LI LU NL SE**

(71) Demandeur: **SANOFI, société anonyme**
**40, Avenue George V**
**F-75008 Paris(FR)**

(72) Inventeur: **Bianchetti, Alberto**
**Via Corridoni 11**
**I-20122 Milano(IT)**

(72) Inventeur: **Nisato, Dino**
**Viale Golgi, 80**
**Pavia(IT)**

(72) Inventeur: **Sacilotto, Roberto**
**Via Sirio 3/L**
**I-20060 Cassina de Pecchi Milano(IT)**

(72) Inventeur: **Manara, Luciano**
**Via Novella 2**
**I-15040 Pietra Marazzi (Allessandria)(IT)**

(74) Mandataire: **Gillard, Marie-Louise et al,**
**Cabinet Beau de Loménie 55, Rue d'Amsterdam**
**F-75008 Paris(FR)**

(54) **Procédé de préparation d'halométhylates de levallorphan et compositions pharmaceutiques antagonistes périphériques des opiacés en contenant.**

(57) Procédé de préparation d'halométhylates de lévallorphan par réaction d'un halogénure de méthyle sur le lévallorphan; produits ainsi obtenus à action antagoniste périphérique des opiacés et compositions pharmaceutiques utiles pour prévenir les effets secondaires des opiacés, notamment la constipation.

EP 0 113 632 A1

Croydon Printing Company Ltd

Procédé de préparation d'halométhylates de lévallorphan et compositions pharmaceutiques antigonistes périphériques des opiacés en contenant.

La présente invention concerne des antagonistes périphériques des opiacés.

Malgré les divers problèmes liés à l'usage des opiacés, tels que la tolérance et la dépendance physique, les médicaments morphiniques restent sans égaux dans le traitement de la douleur violente.

Un effet secondaire, souvent sérieux, des morphiniques est représenté par la constipation qui est dûe à leur action locale au niveau des récepteurs intestinaux des opiacés.

Pour pallier ce type d'effet secondaire très gênant pour le patient, il a été proposé qu'un antagoniste des narcotiques ayant une faible capacité de pénétration à travers la barrière hématoméningée pourrait réduire d'une façon significative la constipation provoquée par les médicaments morphiniques sans en réduire substantiellement les effets analgésiques.

Le brevet US 4 176 186 décrit des dérivés quaternaires d'antagonistes morphiniques qui préviennent ou éliminent les effets secondaires des opiacés sur la motilité intestinale. Parmi les composés revendiqués par le brevet ci-dessus, les composés préférés sont les dérivés quaternaires de la N-allyl-noroxymorphone, ci-après désignée "naloxone".

Un autre composé, le bromoallylate de lévallorphan, a été décrit comme antagoniste périphérique des opiacés (Life Sci. 1982, 31, 2261).

Il a été maintenant trouvé qu'en faisant réagir le lévallorphan avec un halogénure de méthyle, on obtient un composé, ici désigné "halométhylate de lévallorphan", de formule

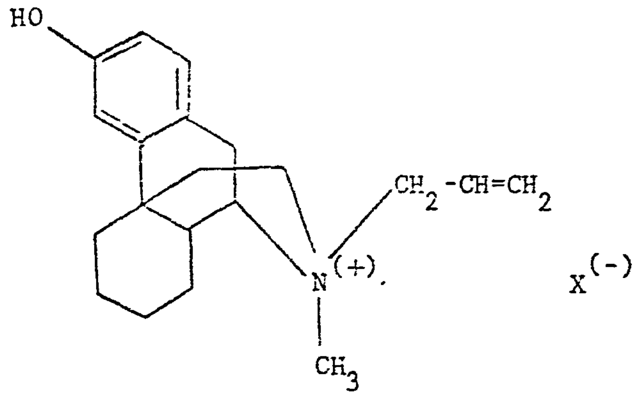

I

dans laquelle X représente le chlore, le brome ou l'iode, qui possède une capacité de pénétration de la barrière hématoméningée pratiquement nulle tout en conservant une très bonne activité antagoniste périphérique.

Le terme "halométhylate de lévallorphan" est utilisé ici pour distinguer le produit préparé comme indiqué ci-dessus de celui obtenu par réaction d'un halogénure d'allyle sur le lévorphanol, ci-après désigné "haloallylate de lévorphanol", qui possède également la formule I ci-dessus, mais des caractéristiques physico-chimiques différentes et qui est inactif sur le plan de l'activité antagoniste, périphérique ou centrale.

En effet, la quaternarisation de l'atome d'azote en position 17 introduit un nouveau centre d'asymétrie et les deux diastéreoisomères peuvent être obtenus par synthèse différentiée comme indiqué ci-dessus en dans J. Med. Chem 1982, 25, 1278-1280 où est décrite la préparation des deux diastéreoisomères dérivés de la nalorphine et de la morphine.

Ainsi la présente invention a pour objet, selon un de ses aspects, un procédé pour la préparation d'un halométhylate de lévallorphan de formule I, caractérisé en ce que :

a) on traite le lévallorphan avec un halogénure de méthyle de formule $CH_3$-X, où X est tel que défini ci-dessus, à une température entre la température ambiante (20°C environ) et 110°C dans une solvant polaire ;

b) on isole le produit ainsi obtenu ; et

c) on soumet ledit produit à des cristallisations successives jusqu'à l'obtention d'un pouvoir rotatoire constant.

Comme solvant polaire on utilise aussi bien un solvant protique, tel qu'un alcool, ou aprotique, tel que le diméthylformamide, l'acétone, le diméthylsulfoxyde et similaires.

La durée de la réaction peut varier entre 2 et 120 heures en fonction de la température de réaction ; en général, après 5-7 heures de chauffage à 70-80°C, la réaction est complète et le produit est isolé selon les techniques classiques, par exemple par précipitation ou par évaporation du solvant, de préférence sous pression réduite à l'aide d'une pompe tournante.

Lorsque, après l'évaporation du solvant, le résidu contient encore des petites quantités de solvant et/ou d'eau, leur élimination complète peut être favorisée en ajoutant un solvant de bas point d'ébullition, de l'acétone ou de l'éthanol absolu de préférence, et en évaporant sous pression réduite.

Le diastéreoisomère halométhylate de lévallorphan est isolé à l'état pur par cristallisations repétées en utilisant un solvant approprié, l'éthanol de préférence, pour éliminer l'autre diastéreoisomère.

Selon un autre de ses aspects, l'invention a pour objet les halométhylates de lévallorphan obtenus en mettant en oeuvre le procédé ci-dessus.

Les halométhylates de lévallorphan, en tant que diastéreoisomères purs, sont des produits nouveaux.

Dans le volume Synthetic Analgesics, Ed. D.H.R. Barton et W. Von Doering, Pergamon Press, 1974, part II, page 48, J. Hellerbach et al. ont indiqué, dans un tableau récapitulatif, un produit de formule I ci-dessus, où X est l'iode, mais aucune référence sur la synthèse chimique du produit n'est indiquée. Ses constantes physicochimiques sont différentes de celles du composé de l'exemple ci-dessous, surtout le point de fusion. Sur le plan de l'activité pharmacologique, une note au bout du tableau indique que le produit est classé parmi ceux ayant une activité analgésique faible ou nulle.

L'antagonisme à l'effet constipant de la morphine des halométhylates de lévallorphan de la présente invention a été évalué selon le test décrit par A.F. Green (Pr. J. Pharmacol.

Chemother. 1959, 14, 26), opportunément modifié.

On utilise trois groupes de souris femelles de 20 g environ à jeun depuis 20 heures. A un groupe témoin, on administre par voie orale un repas constitué par 0,2 ml d'un mélange de gomme arabique 5 % (6 ml), farine (2 g) et charbon (1 g). Au deuxième groupe on administre par la voie sous-cutanée 12 mg/kg de morphine et, immédiatement après, le repas au charbon ci-dessus. Le troisième groupe est traité par l'antagoniste sous examen par voie sous-cutanée, puis, après 5 minutes, par 12 mg/kg de morphine par voie sous-cutanée et, tout de suite après, par le repas au charbon. Après trente minutes, les animaux sont sacrifiés pour l'évaluation de la portion intestinale parcourue par le charbon, exprimée en pour cent de la longueur totale de l'intestin grêle.

L'antagonisme à l'effet analgésique de la morphine a été évalué selon le test classique pour l'analgésie de la plaque chauffante (Eddy et Leibach, J. Pharmacol. 1953, 107, 385, modifié par Janseen et Jagenau, J. Pharm. Pharmacol. 1957, 9, 381-400). Ce test est très sensible pour l'action des antogonistes des opiacés.

On utilise des souris femelles de 18 à 22 g. Les substances sont administrées en solution physiologique (20 ml/kg) par voie sous-cutanée 5 minutes avant l'administration de la morphine (24 mg/kg s.c.). Trente minutes après la morphine, on place les animaux sur la plaque chauffante et l'on évalue le temps de réaction des animaux qui présentent la réaction nocicéptive (léchage des pattes).

L'activité des substances à activité antagoniste périphérique des narcotiques est évaluée sur la base de l'inhibition pour cent de l'effet constipant et de l'effet analgésique de la morphine. La dose qui inhibe de 50 % lesdits effets ($DI_{50}c$ pour l'effet constipant et $DI_{50}a$ pour l'effet analgésique) a été extrapolée de la droite de régression log dose-réponse en appliquant l'analyse de la variance par régression linéaire.

L'activité d'un composé représentatif, le iodométhylate de lévallorphan, a été évaluée en comparaison à celles de son diastéreoisomère, le iodoallylate de lévorphanol, du bromoallylate de lévallorphan cité ci-dessus, d'un antagoniste des narcotiques

bien connu, la naloxone, du sulfométhylate de naloxone décrit dans le brevet US 4 176 186 et du bromométhylate de nalorphine décrit dans la publication parue dans J. Med. Chem. 1982, 25, 1278-1280 déjà citée.

Les résultats sont résumés dans le tableau I ci-dessous.

TABLEAU I

| Composé | Antagonisme à l'effet analgésique | Antagonisme à l'effet constipant | $DI_{50}a/DI_{50}c$ |
|---|---|---|---|
| | $DI_{50}a$ | $DI_{50}c$ | |
| iodométhylate de lévallorphan | 50 | 7,6 mg/kg (4,5 - 13,0) | 6,7 |
| iodoallylate de lévorphanol | 50 | 50 | n.d. |
| bromoallylate de lévallorphan | 15,2 mg/kg (9,7 - 24,0) | 15,6 mg/kg (8,5 - 28,9) | 0,97 |
| sulfométhylate de naloxone | 1,6 mg/kg (0,7 - 3,6) | 5,8 mg/kg (2,9 - 11,5) | 0,27 |
| naloxone | 0,06 mg/kg (0,02 - 0,15) | 0,8 mg/kg (0,5 - 1,2) | 0,08 |
| bromométhylate de nalorphine | 8,6 mg/kg (5,5 - 13,9) | 3,7 mg/kg (1,7 - 8,1) | 2,32 |

De ce tableau il ressort que seulement le iodométhylate de lévallorphan ne passe pas la barrière hématoméningée tandis que le iodoallylate de lévorphanol est inactif et les autres composés traversent, dans une mesure plus ou moins prononcée, la barrière hématoméningée.

Le tableau I montre aussi que le iodométhylate de lévallorphan de la présente invention a une activité plus de 2 fois supérieure à celle du bromoallylate de lévallorphan dans le test de l'antagonisme à l'effet constipant de la morphine, et que le produit connu, dans un test d'analgésie très sensible, montre une tendance au passage de la barrière hématoméningée.

Il en découle que le composé représentatif de la présente invention ne manifeste pas d'effet central se montrant ainsi un antagoniste périphérique partiquement pur, alors que les composés de référence qui se sont montrés actifs ont également une activité centrale plus ou moins évidente.

Les composés de la présente invention sont peu toxiques et ils sont donc utiles comme médicaments.

Ainsi, la présente invention a pour objet, selon autre aspect, des compositions pharmaceutiques à action antagoniste périphérique des opiacés renfermant, en tant qu'ingrédient actif, un halométhylate de lévallorphan en mélange avec un excipient pharmaceutique.

Les compositions pharmaceutiques à action antagoniste périphérique des opiacés de la présente invention sont utiles dans le traitement des conditions pathologiques où il existe des taux altérés d'opiacés exogènes ou endogènes, ou une hypersensibilité aux opiacés hors du système nerveux central des mammifères.

Ainsi, les compositions de la présente invention peuvent être administrées aux mammifères, animaux et êtres humains, avec les opiacés afin d'en prévenir les effets secondaires, notamment la constipation, qui dérivent notamment de l'activation des récepteurs des opiacés situés à la périphérie, sans compromettre l'analgésie, ou n'importe quelle autre action des opiacés, provoquée par stimulation des récepteurs centraux de la part de l'opiacé.

Afin d'obtenir l'effet antagoniste périphérique désiré, la dose de principe actif peut varier entre 0,05 et 200 mg par kg de poids du corps et par jour.

Chaque dose unitaire peut contenir de 1 à 500 mg d'ingrédient actif en combinaison avec un support pharmaceutique. Cette dose unitaire peut être administrée aux mammifères 1 à 4 fois par jour.

Les compositions pharmaceutiques de la présente invention à action antagoniste périphérique des opiacés peuvent être formulées pour l'administration orale, sublinguale, nasale, sous-cutanée, intramusculaire, intraveineuse, transdermique ou rectale en mélangeant l'ingrédient actif de formule I ci-dessus avec des supports

pharmaceutiques classiques.

Les formes unitaires d'administration comprennent les comprimés, les gélules, les poudres, les granules et les solutions ou suspensions orales, les suppositoires ainsi que les ampoules utiles pour une administration parentérale.

Lorsqu'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter d'une autre manière de telle sorte qu'ils aient une activité prolongée ou retardée et qu'ils libèrent continuellement une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir peut contenir l'ingrédient actif avec un édulcorant éventuellement hypocalorique, du méthylparaben et du propylparaben comme antiseptiques, ainsi qu'un agent donnant du goût et un colorant approprié.

Les poudres ou les granulats dispersibles dans l'eau peuvent contenir l'ingrédient actif en mélange avec des agents de dispersion ou des agents mouillants, ou des agents de mise en suspension, tels que la polyvinylpyrrolidone et similaires, de même qu'avec des édulcorants ou des correcteurs du goût.

Pour une application rectale, on a recours à des suppositoires qui sont préparés avec des liants fondant à la température rectale, par exemple du beurre de cacao ou des polyéthylèneglycols.

Pour une administration orale en gouttes ou pour une administration parentérale, on utilise des suspensions aqueuses, des solutions salines isotoniques ou des solutions particulières, stériles et injectables qui contiennent des agents de dispersion et/ou des mouillants pharmacologiquement compatibles, par exemple le propylèneglycol ou le butylèneglycol.

Le principe actif peut être formulé également sous forme de microcapsules, éventuellement avec un ou plusieurs supports ou additifs.

Les compositions de la présente invention peuvent contenir, à côté de l'halométhylate de lévallorphan, d'autres principes actifs tels que, par exemple, des analgésiques narcotiques comme la morphine, la codéine, la buprénorphine et similaires, des antitussifs ou d'autres médicaments appropriés.

Les exemples suivants illustrent l'invention sans toutefois la limiter.

PREPARATION

L'iodoallylate de lévorphanol, utilisé comme produit de référence dans les essais pharmacologiques décrits ci-dessus, a été préparé de la façon suivante : on agite 24 heures à l'abri de la lumière et à la température ambiante un mélange de 6 g de lévorphanol, 50 ml de méthanol et 20 ml d'iodure d'allyle, puis on évapore à sec sous pression réduite. On reprend le résidu huileux par de l'acétone et on agite le mélange 1 heure à la température ambiante. Le solide qui se forme est filtré et cristallisé trois fois dans de l'éthanol à 95 % jusqu'à l'obtention d'un $[\alpha]_D^{20}$ constant. On obtient ainsi 4 g d'iodoallylate de lévorphanol ; p.F. 230°C (déc.), $[\alpha]_D^{20}$ = -33,9° (méthanol, c = 0,2 %).

EXEMPLE 1

On chauffe 6 heures sous agitation à la température externe de 80°C un mélange de 5 g de lévallorphan, 70 ml de diméthylformamide et 6,7 g d'iodure de méthyle, puis on concentre sous pression réduite tout en éliminant le solvant et l'excès d'alkylant par addition d'acétone. Par refroidissement de la solution acétonique on obtient un solide qui, après filtration et séchage sous pression réduite, fond à 225-227°C. Par recristallisation dans 50 ml d'éthanol à 90 %, on obtient 5 g d'un produit blanc ; point de fusion 230 à 232°C. Après deux autres cristallisations jusqu'à l'obtention d'un pouvoir rotatoire constant, on obtient, avec un rendement de 40%, l'iodométhylate de lévallorphan pur ; point de fusion 232 à 234°C (méthode de Tottoli) ; $[\alpha]_D^{20}$ = -64° ± 1° (méthanol, c = 0,2 %).

Le spectre protonique RMN effectué à 250 MHz dans du diméthylsulfoxyde deutérié, en utilisant le tétraméthylsilane en

tant que standard interne, présente un singulet à 3,20 $\pm$ 0,02 ppm, relatif au groupe méthyle en position 17.

L'autre diastéreoisomère, décrit dans la préparation, présente le même singulet à 3,04 $\pm$ 0,02 ppm.

EXEMPLE 2

On prépare des comprimés ayant la composition suivante :

| | |
|---|---|
| iodométhylate de lévallorphan | 50 mg |
| lactose | 145 mg |
| avicel | 100 mg |
| stéarate de magnésium | 5 mg |

en broyant l'ingrédient actif jusqu'à une dimension de particules de 0,4 mm, en le faisant passer à travers un tamis de 0,4 mm d'ouverture de maille, en mélangeant la matière broyée avec les autres constituants et en comprimant pour former les comprimés.

De la même façon, on prépare des comprimés contenant 40 mg de iodométhylate de lévallorphan.

EXEMPLE 3

On prépare des gélules à base d'iodométhylate de lévallorphan ayant la composition suivante :

| | |
|---|---|
| iodométhylate de lévallorphan | 15 mg |
| lactose | 120 mg |
| stéarate de magnésium | 5 mg |

en mélangeant intimement les ingrédients ci-dessus et en versant le mélange dans des gélules de gélatine dure

De la même façon, on prépare des gélules contenant 25 mg de iodométhylate de lévallorphan.

EXEMPLE 4

10 000 gélules ayant une teneur en substance active de 50 mg sont préparées à partir des constituants suivants : 500 g d'iodométhylate de lévallorphan, 495 g de cellulose microcristalline, 5 g de gel de silice amorphe. Les constituants ci-dessus sont bien mélangés et on les introduit dans des gélules de gélatine dure de dimension 4.

EXEMPLE 5

On prépare une solution aqueuse stérile appropriée pour une utilisation parentérale en ampoules ayant la composition suivante :

| | | |
|---|---|---|
| iodométhylate de lévallorphan | | 10 mg |
| eau pour préparation injectable | q.s.p | 2 ml |

EXEMPLE 6

On prépare des suppositoires ayant la composition suivante :

| | | |
|---|---|---|
| iodométhylate de lévallorphan | | 50 mg |
| lactose | | 250 mg |
| masse pour suppositoires | q.s.p. | 1,7 g |

On mélange le iodométhylate de lévallorphan avec le lactose et on met le mélange uniformément en suspension dans la masse pour suppositoires fondue.

On verse la suspension dans des moules refroidis pour former des suppositoires d'un poids de 1,7 g.

EXEMPLE 7

On prépare des comprimés dragéifiés contenant chacun 30 mg d'iodométhylate de lévallorphan en utilisant comme excipient du talc, du lactose, de l'amidon de maïs, de l'alginate de sodium, du sucre semoule, du sucre cristallisé, du stéarate de magnésium, de la gomme laque blanche, de la gélatine alimentaire, de l'érythrosine, du bioxyde de titane et de la cire blanche.

## REVENDICATIONS

1. Procédé pour la préparation d'un halométhylate de lévallorphan de formule

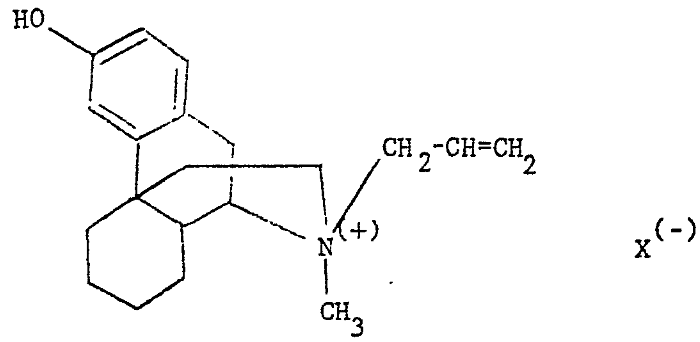

dans laquelle X représente le chlore, le brome ou l'iode, caractérisé en ce que :

a) on traite le lévallorphan avec un halogénure de méthyle de formule $CH_3 - X$, ou X est tel que défini ci-dessus, à une température entre la température ambiante et 110°C dans un solvant polaire ;

b) on isole le produit ainsi obtenu ; et

c) on soumet ledit produit à des cristallisations successives jusqu'à l'obtention d'un pouvoir rotatoire constant.

2. Halométhylate de lévallorphan, tel qu'obtenu par le procédé selon la revendication 1.

3. Iodométhylate de lévallorphan ayant les caractéristiques physico-chimiques suivantes :

$[\alpha]_D^{20} = -64° \pm 1°$ (méthanol, c = 0,2 %)

spectre 1H-RMN (DMSO-$d_6$) : singulet à 3,20 ± 0,02 ppm.

4. Composition pharmaceutique à action antagoniste périphérique des opiacés, caractérisée en ce qu'elle contient, en tant que principe actif, un halométhylate de lévallorphan de formule

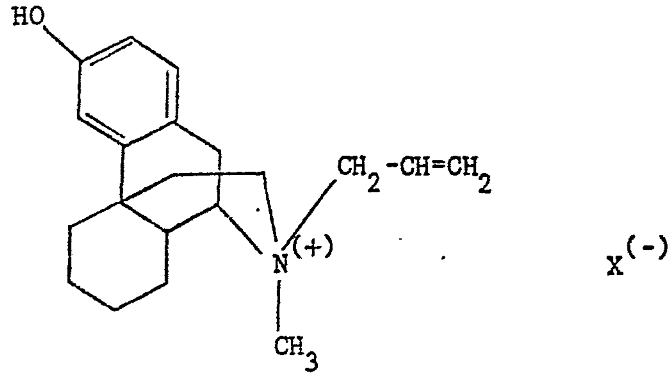

dans laquelle X représente le chlore, le brome ou l'iode.

5. Composition pharmaceutique à action antagoniste périphérique des opiacés, caractérisée en ce qu'elle contient, en tant que principe actif, un composé selon l'une des revendications 2 ou 3.

6. Composition pharmaceutique selon l'une des revendications 4 ou 5, caractérisé en ce qu'elle est sous forme d'unité de dosage, et contient 1 à 500 mg de principe actif en mélange avec un excipient pharmaceutique.

7. Composition pharceutique selon l'une quelconque des revendications 4 à 6, caractérisée en ce que le principe actif est l'iodométhylate de lévallorphan.

Office européen des brevets

# RAPPORT DE RECHERCHE EUROPEENNE

0113632

Numéro de la demande

EP 83 40 2535

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| X | CHEMICAL ABSTRACTS REGISTRY HANDBOOK, no. section, 1977, supplément, page 1827 RF, réf. 63868-46-2<br>* Page 1827 RF, colonne de gauche * & LIFE SCI. 1983, 33 (suppl. 1) 415-18 (Cat. P,X) | 1-7 | C 07 D 221/28<br>A 61 K 31/435 |
| X | CHEMICAL ASEBSTRACTS REGISTRY HANDBOOK<br>& LIFE SCI. 1983, 33 (suppl. 1) 481-4 (Cat. P,X)<br>--- | 3-7 | |
| A | HELVETICA CHIMICA ACTA, vol. 39, no. 49, fasciculus II, 1956, pages 429-440, Bale, CH<br>J. HELLERBACH et al.: "(-)-3-Hydroxy-N-allyl-morhpinan und verwandte Verbindungen" * Page 429, formule II, pages 433-434 *<br>--- | 1,4-7 | **DOMAINES TECHNIQUES RECHERCHES (Int. Cl. 3)** |
| D,A | US-A-4 176 186 (L.I. GOLDBERG)<br>* En entier *<br>--- | 1-7 | C 07 D 221/00<br>A 61 K 31/00 |
| A | FR-A-1 179 915 (HOFFMANN-LA ROCHE)<br>* Exemple 5 *<br>--- -/- | 1,4-7 | |

Le présent rapport de recherche a été établi pour toutes les revendications

| Lieu de la recherche | Date d'achevement de la recherche | Examinateur |
|---|---|---|
| LA HAYE | 04-03-1984 | NUYTS A.M.K.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82

Office européen des brevets

**RAPPORT DE RECHERCHE EUROPEENNE**

0113632
Numéro de la demande

EP 83 40 2535

Page 2

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| D,A | JOURNAL OF MEDICINAL CHEMISTRY, vol. 25, novembre 1982, pages 1278-1280, American Chemical Society, Columbus, Ohio, US R.J. KOBYLECKI et al.: "N-Methylnalorphine: Definition of N-allyl conformation for antagonism at the opiate receptor" * En entier *<br>----- | 3-7 | |

DOMAINES TECHNIQUES RECHERCHES (Int. Cl. 3)

Le présent rapport de recherche a été établi pour toutes les revendications

| Lieu de la recherche | Date d'achèvement de la recherche | Examinateur |
|---|---|---|
| LA HAYE | 04-03-1984 | NUYTS A.M.K.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention
E : document de brevet antérieur, mais publié à la date de dépôt ou après cette date
D : cité dans la demande
L : cité pour d'autres raisons
& : membre de la même famille, document correspondant